# EUROPEAN PATENT APPLICATION

(11) **EP 1 921 444 A1**
(43) Date of publication of application: **14.05.2008**
(21) Application number: 07119696.8
(22) Date of filing: 31.10.2007
(51) Int. Cl.: G01N 27/407, G01N 33/00

(54) **Exhaust gas sensors and methods for measuring concentrations of nox and ammonia and temperatures of the sensors**

(30) Priority: 09.11.2006 US 558123
(71) Applicant: Delphi Technologies, Inc., Troy, Michigan 48007 (US)
(72) Inventor: WANG, Da Yu, Troy, MI 48084 (US); FARHAT, Robert, Grosse Pte Park, MI 48230 (US)
(74) Representative: Denton, Michael John

(57) **Abstract**

Exhaust gas sensors and methods for measuring concentrations of NOx and ammonia and temperatures of the sensors are provided. In one exemplary embodiment, an exhaust gas sensor (12) utilizes one zirconia layer (28) and a plurality of alumina layers therein for generating signals indicative of concentrations of NOx and ammonia, and having an impedance indicative of a temperature of the exhaust gas sensor.

## Description

### TECHNICAL FIELD

This application relates to exhaust gas sensors and methods for measuring concentrations of NOx and ammonia and temperatures of the sensors.

### BACKGROUND OF THE INVENTION

An exhaust gas sensor has been developed that can measure a concentration of an exhaust gas constituent and a temperature of the sensor. However, the exhaust gas sensor utilizes a plurality of zirconia layers. The inventors herein have recognized that it is undesirable to have the plurality of zirconia layers because the zirconia layers can have a dielectric breakdown which can reduce the operational life of the exhaust gas sensor.

Accordingly, the inventor herein have recognized a need for an improved exhaust gas sensor which utilizes only one zirconia layer to measure exhaust gas constituents and a temperature of the exhaust gas sensor.

### SUMMARY OF THE INVENTION

An exhaust gas sensor in accordance with an exemplary embodiment is provided. The exhaust gas sensor includes only one zirconia layer having a first side and a second side opposite the first side. The exhaust gas sensor further includes a NOx sensing electrode coupled to the first side of the zirconia layer. The exhaust gas sensor further includes an ammonia sensing electrode coupled to the first side of the zirconia layer. The exhaust gas sensor further includes a platinum electrode coupled to the second side of the zirconia layer. The platinum electrode is electrically grounded.
The NOx sensing electrode, the ammonia sensing electrode, and the platinum electrode are configured to communicate with exhaust gases. A first voltage between the NOx sensing electrode and the platinum electrode is indicative of a concentration of NOx, and a second voltage between the ammonia sensing electrode and the platinum electrode is indicative of a concentration of ammonia. Further, an impedance between the NOx sensing electrode and the platinum electrode is indicative of a temperature of the exhaust gas sensor.

A method for measuring concentrations of both NOx and ammonia and a temperature utilizing an exhaust gas sensor in accordance with another exemplary embodiment is provided. The exhaust gas sensor has only one zirconia layer having a first side and a second side opposite the first side. The exhaust gas sensor further includes a NOx sensing electrode coupled to the first side of the zirconia layer, an ammonia sensing electrode coupled to the first side of the zirconia layer, and a platinum electrode coupled to the second side of the zirconia layer. The platinum electrode is electrically grounded. The NOx sensing electrode, the ammonia sensing electrode, and the platinum electrode are configured to communicate with exhaust gases. The method includes measuring a first voltage between the NOx sensing electrode and the platinum electrode that is indicative of the concentration of NOx. The method further includes measuring a second voltage between the ammonia sensing electrode and the platinum electrode that is indicative of the concentration of ammonia. The method further includes, after measuring the first voltage and the second voltage, measuring an impedance between the NOx sensing electrode and the platinum electrode that is indicative of the temperature of the exhaust gas sensor.

A method for measuring concentrations of both NOx and ammonia and a temperature utilizing an exhaust gas sensor in accordance with another exemplary embodiment is provided. The exhaust gas sensor has only one zirconia layer having a first side and a second side opposite the first side, a NOx sensing electrode coupled to the first side of the zirconia layer, an ammonia sensing electrode coupled to the first side of the zirconia layer, and a platinum electrode coupled to the second side of the zirconia layer. The platinum electrode is electrically grounded. The NOx sensing electrode, the ammonia sensing electrode, and the platinum electrode are configured to communicate with exhaust gases.
The method includes measuring a first voltage between the NOx sensing electrode and the platinum electrode that is indicative of the concentration of NOx. The method further includes measuring a second voltage between the ammonia sensing electrode and the platinum electrode that is indicative of the concentration of ammonia. The method further includes, after measuring the first voltage and the second voltage, measuring an impedance between the ammonia sensing electrode and the platinum electrode that is indicative of the temperature of the exhaust gas sensor.

An exhaust gas sensor in accordance with another exemplary embodiment is provided. The exhaust gas sensor includes only one zirconia layer having a first side and a second side opposite the first side. The exhaust gas sensor further includes a NOx sensing electrode coupled to the first side of the zirconia layer. The exhaust gas sensor further includes an ammonia sensing electrode coupled to the first side of the zirconia layer. The exhaust gas sensor further includes a platinum electrode coupled to the second side of the zirconia layer. The platinum electrode is electrically grounded.
The NOx sensing electrode, the ammonia sensing electrode, and the platinum electrode are configured to communicate with exhaust gases. The exhaust gas sensor further includes an impedance sensing electrode coupled to the first side of the zirconia layer. A first voltage between the NOx sensing electrode and the platinum electrode is indicative of a concentration of NOx, and a second voltage between the ammonia sensing electrode and the platinum electrode is indicative of a concentration of ammonia, and an impedance between the impedance sensing electrode and the platinum electrode is indicative of a temperature of the exhaust gas sensor.

A method for measuring concentrations of both NOx and ammonia and a temperature utilizing an exhaust gas sensor in accordance with another exemplary embodiment is provided. The exhaust gas sensor has only one zirconia layer having a first side and a second side opposite the first side. The exhaust gas sensor further includes a NOx sensing electrode coupled to the first side of the zirconia layer, an ammonia sensing electrode coupled to the first side of the zirconia layer, and a platinum electrode coupled to the second side of the zirconia layer. The platinum electrode is electrically grounded. The NOx sensing electrode, the ammonia sensing electrode, and the platinum electrode are configured to communicate with exhaust gases. The exhaust gas sensor further includes an impedance sensing electrode coupled to the first side of the zirconia layer. The method includes measuring a first voltage between the NOx sensing electrode and the platinum electrode that is indicative of the concentration of NOx. The method further includes measuring a second voltage between the ammonia sensing electrode and the platinum electrode that is indicative of the concentration of ammonia. The method further includes, after measuring the first voltage and the second voltage, measuring an impedance between the impedance sensing electrode and the platinum electrode that is indicative of the temperature of the exhaust gas sensor.

### BRIEF DESCRIPTION OF THE DRAWINGS

Figure 1 is a schematic of an exhaust gas monitoring system having an exhaust gas sensor, a voltage source, an impedance measurement circuit, and a controller in accordance with an exemplary embodiment;
Figure 2 is a schematic of a portion of the exhaust gas sensor in the exhaust gas monitoring system of Figure 1;
Figure 3 is an electrical schematic of the impedance measurement circuit in the exhaust gas monitoring system of Figure 1;
Figure 4 is a signal a schematic illustrating signals generated by the impedance measurement circuit of Figure 3;
Figure 5 is a flowchart of a method for measuring concentrations of NOx and ammonia, and a temperature of an exhaust gas sensor in accordance with another exemplary embodiment;
Figure 6 is a flowchart of a method for measuring concentrations of NOx and ammonia, and a temperature of an exhaust gas sensor in accordance with another exemplary embodiment;
Figure 7 is a schematic of an exhaust gas monitoring system having an exhaust gas sensor, a voltage source, an impedance measurement circuit, and a controller in accordance with another exemplary embodiment;
Figure 8 is a flowchart of a method for measuring concentrations of NOx and ammonia, and a temperature of an exhaust gas sensor in accordance with another exemplary embodiment;
Figure 9 is a schematic of an exhaust gas monitoring system having an exhaust gas sensor, a voltage source, an impedance measurement circuit, and a controller in accordance with another exemplary embodiment.

### DESCRIPTION OF THE PREFERRED EMBODIMENTS

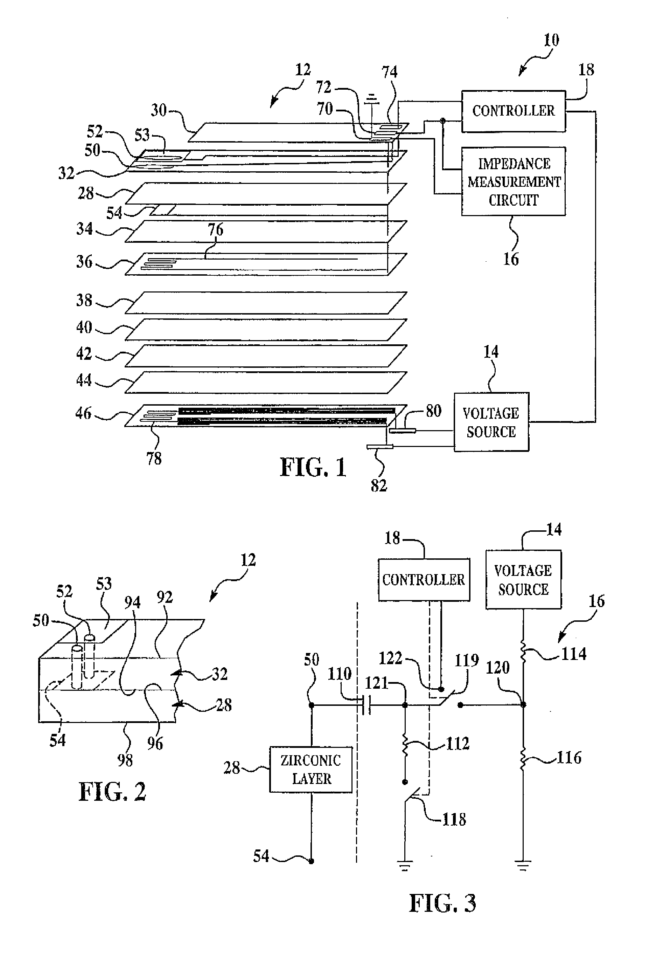

Referring to Figure 1, an exhaust gas monitoring system 10 for determining concentrations of NOx and ammonia in exhaust gases communicating with the exhaust gas sensor 12, and for determining a temperature of the exhaust gas sensor 12 is illustrated. The exhaust gas monitoring system 10 includes the exhaust gas sensor 12, a voltage source 14, an impedance measurement circuit 16, and a controller 18.

The exhaust gas sensor 12 is provided to generate a voltage indicative of a NOx concentration in exhaust gases communicating with the exhaust gas sensor 12. The exhaust gas sensor 12 is further configured to generate another voltage indicative of a detected ammonia concentration in exhaust gases communicating with the exhaust gas sensor 12. Further, an impedance of a zirconia layer of the exhaust gas sensor 12 is indicative of a temperature of the exhaust gas sensor 12. The exhaust gas sensor 12 includes a zirconia layer 28, alumina layers 30, 32, 34, 36, 38, 40, 42, 44, 46, a NOx sensing electrode 50, an ammonia sensing electrode 52, a platinum pad 53, a platinum electrode 54, electrodes 70, 72, 74, an electromagnetic (EM) shield 76, a heater coil 78, and electrodes 80, 82.

Referring to Figures 1 and 2, the NOx sensing electrode 50 extends through the alumina layer 32 from a surface 92 to a surface 94 thereof. As shown, the electrode 50 contacts the surface 96 of the zirconia layer 28. The NOx sensing electrode 50 is further electrically coupled to the electrode 72 on the alumina layer 30. The ammonia sensing electrode 52 extends through the platinum pad 53 on the alumina layer 32 and further through the alumina layer 32 from the surface 92 to the surface 94 thereof. As shown, the ammonia sensing electrode 52 contacts the surface 96 of the zirconia layer 28. The ammonia sensing electrode 52 is further electrically coupled to the electrode 74 on the alumina layer 30. The reference platinum electrode 54 is disposed on the surface 98 of the zirconia layer 28 and is further electrically coupled to the electrode 70. The electrode 70 is electrically coupled to electrical ground. The platinum electrode 54 communicates with an aperture (not shown) in the zirconia layer 28 that further communicates with exhaust gases. Further, the NOx sensing electrode 50 and the ammonia sensing electrode 52 communicate with the exhaust gases. The combination of the NOx sensing electrode 50, the zirconia layer 28, and the platinum electrode 54 form an electrochemical cell which generates a voltage indicative of a concentration of NOx in exhaust gases communicating with the exhaust gas sensor 12. The combination of the ammonia sensing electrode 52, the zirconia layer 28, and the platinum electrode 54 form an electrochemical cell which generates a voltage indicative of a concentration of ammonia in exhaust gases communicating with the exhaust gas sensor 12. In one exemplary embodiment, the NOx sensing electrode 50 is constructed from TbCrO3 (doped with 5% MgO). Of course in alternative embodiments, other materials known to those skilled in the art could be utilized to construct the NOx sensing electrode 50. In one exemplary embodiment, the ammonia sensing electrode 52 is constructed from BiVO4 (doped with 5% MgO). Of course in alternative embodiments, other materials known to those skilled in the art could be utilized to construct the ammonia sensing electrode 52.

The alumina layer 30 is disposed adjacent a top side of the alumina layer 32. The alumina layer 30 has the electrodes 70, 72, 74 disposed thereon. The electrodes 72, 74 are further electrically coupled to the impedance measurement circuit 16 and to the controller 18.

The zirconia layer 28 is disposed against a bottom surface of the alumina layer 32. In one exemplary embodiment, the zirconia layer 28 is constructed from a combination of zirconia, alumina, and ytteria. Of course, other combinations of zirconia and other materials know to those skilled in the art could be utilized in other alternative embodiments.

The alumina layer 34 is disposed against a bottom surface of the zirconia layer 28. Similarly, the alumina layer 36 is disposed against a bottom surface of the alumina layer 34.

The EM shield 76 is disposed on a top surface of the alumina layer 36 and is electrically coupled to electrode 70 an alumina layer 30 which is further electrically coupled to electrical ground. The EM shield 76 is provided to shield the exhaust gas sensor 12 from undesirable electromagnetic noise.

The alumina layer 38 is disposed against a bottom surface of the alumina layer 36. The alumina layer 40 is disposed against a bottom surface of the alumina layer 38. The alumina layer 42 is disposed against a bottom surface of the alumina layer 40. The alumina layer 44 is disposed against a bottom surface of the alumina layer 42. Further, the alumina layer 46 is disposed against a bottom surface of the alumina layer 44.

The heater coil 78 is disposed of a top surface of the alumina layer 46 and is provided to generate heat in response to receiving a voltage from the voltage source 14. In particular, the heater coil 78 is electrically coupled to electrodes 80, 82 which are further electrically coupled to the voltage source 14.

Referring to Figures 1 and 3, the impedance measurement circuit 16 is provided to measure an impedance of the zirconia layer 28 which is indicative of a temperature of the zirconia layer 28 and further indicative of a temperature of the exhaust gas sensor 12. The impedance measurement circuit 16 includes a capacitor 110, resistors 112, 114, 116, and solid-state switches 118, 119. In one exemplary embodiment, the circuit 16 is electrically coupled to the NOx sensing electrode 50 and the platinum electrode 54 to measure the impedance of the exhaust gas sensor 12. In particular, the capacitor 110 is electrically coupled between the platinum electrode 50 and the node 121. Further, the resister 112 and a solid-state switch 118 are electrically coupled its series between the node 121 and electrical ground. The electrical switch 119 is electrically coupled between the nodes 120, 121, and 122. During operation, the switch 119 has a first closed operational position to electrically couple the node 120 to the node 121. Further, the switch 119 has a second closed operational position to electrically couple the node 122 to the node 121. Further, the switch has an open operational position. Further, the resister 114 is electrically coupled between the voltage source 14 and the node 120. Further, the resister 116 is electrically coupled between the node 120 and electrical ground. The operation of the impedance measurement circuit 16 will be explained below.

The controller 18 is provided to measure a voltage between the NOx sensing electrode 50 and the platinum electrode 54 in order to calculate a NOx concentration. The controller 18 is further provided to measure a voltage between the ammonia sensing electrode 52 and the platinum electrode 54 in order to calculate an ammonia concentration. During operation, after the controller 14 has measured voltages indicative of a NOx concentration and an ammonia concentration, the controller 14 generates a control signal to induce the solid-state switch 119 to have the first closed operational position to apply a voltage level Vinitial to the capacitor 110. Thereafter, the controller 18 generates control signals to induce the solid state switch 119 to have the second closed operational position and the solid-state switch 118 to have a closed operational position. Thereafter, the controller 18 measures a voltage at the node 122 that is indicative of the impedance of the zirconia layer 28. In particular, the controller 18 utilizes the following equation to calculate the impedance of the zirconia layer 28: impedance = resistance of resister 112 * (Vinitial-Vfinal) /Vfinal, wherein Vfinal corresponds to a voltage at the node 122 a predetermined time interval after the switch 119 has the second closed operational position. The controller 18 also generates a control voltage to induce the voltage source 14 to output a desired power level, based on the calculated temperature, in order to cause the heater coil 78 to maintain the exhaust gas sensor within a desired temperature range.

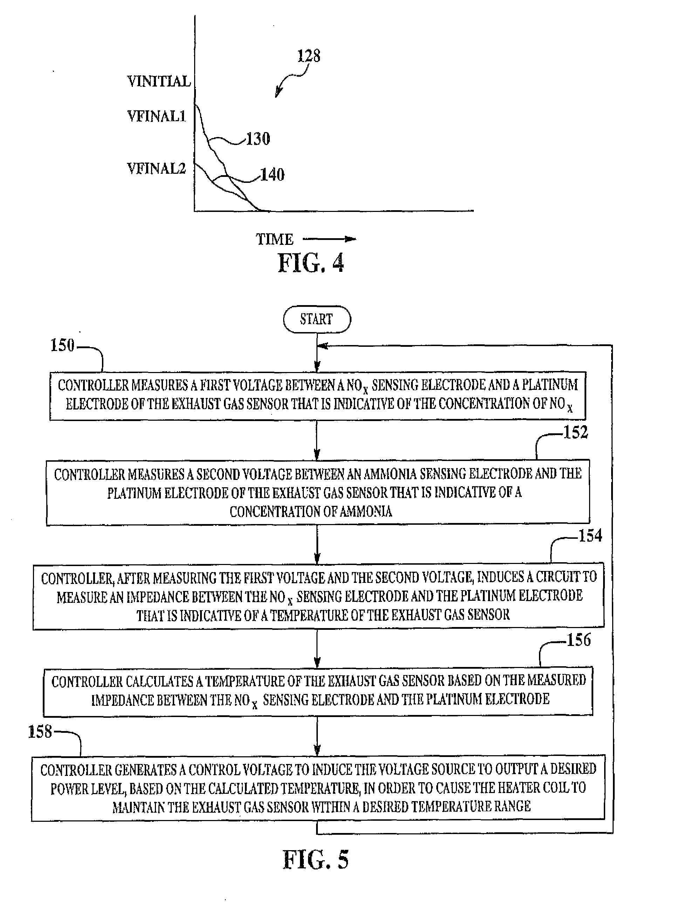

Referring to Figure 4, a graph 128 is illustrated having signal curves 130 and 140. The curve 130 corresponds to the Vfinal voltage over time when the zirconium layer 28 has a first temperature level. The curve 140 corresponds to the Vfinal voltage over time when the zirconium layer 28 has a second temperature level less than the first temperature level.

Referring to Figure 5, a method for determining concentrations of NOx and ammonia in exhaust gases communicating with the exhaust gas sensor 12, and determining a temperature of the exhaust gas sensor 12 will now be explained.

At step 150, the controller 18 measures a first voltage between the NOx sensing electrode 50 and the platinum electrode 54 of the exhaust gas sensor 12 that is indicative of a concentration of NOx. The controller 18 calculates a NOx concentration value based on the first voltage.

At step 152, the controller 18 measures a second voltage between the ammonia sensing electrode 52 and the platinum electrode 54 of the exhaust gas sensor 12 that is indicative of a concentration of ammonia. The controller 18 calculates an ammonia concentration value based on the second voltage.

At step 154, the controller 18, after measuring the first voltage and the second voltage, induces the impedance measurement circuit 16 to measure an impedance between the NOx sensing electrode 50 and the platinum electrode 54 that is indicative of a temperature of the exhaust gas sensor 12.

At step 156, the controller 18 calculates a temperature of the exhaust gas sensor 12 based on the measured impedance between the NOx sensing electrode 50 and the platinum electrode 54.

At step 158, the controller 18 generates a control voltage to induce the voltage source 14 to output a desired power level, based on the calculated temperature, in order to cause the heater coil 78 to maintain the exhaust gas sensor 12 within a desired temperature range. After step 158, the method returns to step 150.

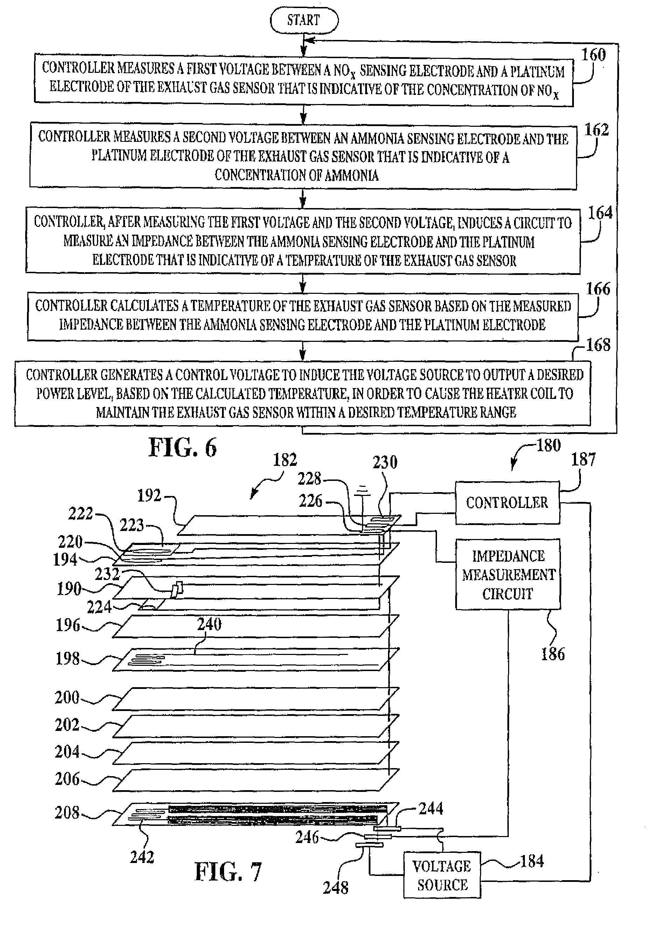

Referring to Figure 6, a method for determining concentrations of NOx and a temperature of the exhaust gas sensor 12 will now be explained.

At step 160, the controller 18 measures a first voltage between the NOx sensing electrode 50 and the platinum electrode 54 of the exhaust gas sensor 12 that is indicative of a concentration of NOx. The controller 18 calculates a NOx concentration value based on the first voltage.

At step 162, the controller 18 measures a second voltage between the ammonia sensing electrode 52 and the platinum electrode 54 of the exhaust gas sensor 12 that is indicative of a concentration of ammonia. The controller 18 calculates an ammonia concentration value based on the second voltage.

At step 164, the controller 18, after measuring the first voltage and the second voltage, induces the impedance measurement circuit 16 to measure an impedance between the ammonia sensing electrode 52 and the platinum electrode 54 that is indicative of a temperature of the exhaust gas sensor 12.

At step 166, the controller 18 calculates a temperature of the exhaust gas sensor 12 based on the measured impedance between the ammonia sensing electrode 52 and the platinum electrode 54.

At step 168, the controller 18 generates a control voltage to induce the voltage source 14 to output a desired power level, based on the calculated temperature, in order to cause the heater coil 78 to maintain the exhaust gas sensor 12 within a desired temperature range. After step 168, the method returns to step 160.

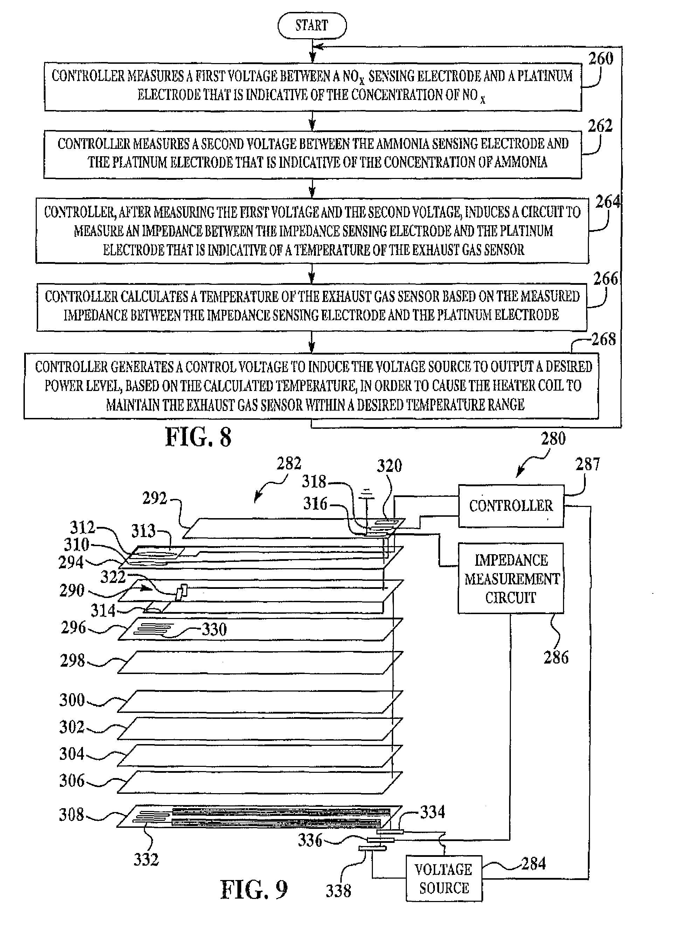

Referring to Figure 7, an exhaust gas monitoring system 180 for determining concentrations of NOx and ammonia, and a temperature of the exhaust gas sensor 182 is illustrated. The exhaust gas monitoring system 180 includes the exhaust gas sensor 182, a voltage source 184, an impedance measurement circuit 186, and a controller 187.

The exhaust gas sensor 182 is provided to generate a voltage indicative of a detected NOx concentration in exhaust gases communicating with the exhaust gas sensor 182. The exhaust gas sensor 182 is further configured to generate another voltage indicative of a detected ammonia concentration in exhaust gases communicating with the exhaust gas sensor 182. Further, an impedance of a zirconia layer of the exhaust gas sensor 182 is indicative of a temperature of the exhaust gas sensor 182. The exhaust gas sensor 182 includes a zirconia layer 190, alumina layers 192, 194, 196, 198, 200, 202, 204, 206, 208, a NOx sensing electrode 220, an ammonia sensing electrode 222, a platinum pad 223, a platinum electrode 224, electrodes 226, 228, 230, 232, an EM shield 240, a heater coil 242, and electrodes 244, 246, and 248.

The NOx sensing electrode 220 extends through the alumina layer 194 and contacts the zirconia layer 190. The NOx sensing electrode 220 is further electrically coupled to the electrode 228 on the alumina layer 192. The ammonia sensing electrode 222 extends through the platinum pad 223 and the alumina layer 194 and contacts the zirconia layer 190. The ammonia sensing electrode 222 is further electrically coupled to the electrode 230 on the alumina layer 192. The platinum electrode 224 is disposed on a bottom surface of the zirconia layer 190 and is further electrically coupled to electrode 226. The electrode 226 is electrically coupled to electrical ground. The platinum electrode 224 communicates with an aperture (not shown) in the zirconia layer 190 that further communicates with exhaust gases. Further, the NOx sensing electrode 220 and the ammonia sensing electrode 222 communicate with the exhaust gases. The combination of the NOx sensing electrode 220, the zirconia layer 190, and the platinum electrode 224 form an electrochemical cell which generates a voltage indicative of a concentration of NOx in exhaust gases communicating with the exhaust gas sensor 182. The combination of the ammonia sensing electrode 222, the zirconia layer 190, and the platinum electrode 224 form an electrochemical cell which generates a voltage indicative of a concentration of ammonia in exhaust gases communicating with the exhaust gas sensor 182. In one exemplary embodiment, the NOx sensing electrode 220 is constructed from TbCrO3 (doped with 5% MgO). Of course in alternative embodiments, other materials known to those skilled in the art could be utilized to construct the NOx sensing electrode 220. In one exemplary embodiment, the ammonia sensing electrode 222 is constructed from BiVO4 (doped with 5% MgO). Of course in alternative embodiments, other materials known to those skilled in the art could be utilized to construct the ammonia sensing electrode 222.

The alumina layer 192 is disposed adjacent a top side of the alumina layer 194. The alumina layer 192 has electrodes 226, 228, 230 disposed thereon. The electrode 226 is further electrically coupled to the impedance measurement circuit 186. The electrodes 228, 230 are electronically coupled to the controller 187.

The zirconia layer 190 is disposed against a bottom surface of the alumina layer 194. In one exemplary embodiment, the zirconia layer 190 is constructed from a combination of zirconia, alumina, and ytteria. Of course, other combinations of zirconia and other materials know to those skilled in the art could be utilized in other alternative embodiments. The platinum temperature sensing electrode 232 is disposed on a top surface of the zirconia layer 190 and is electrically coupled to electrode 246. The platinum temperature sensing electrode 232 communicates with exhaust gases through an aperture between the layer 194 and the layer 190.

The alumina layer 196 is disposed against a bottom surface of the zirconia layer 190. Similarly, the alumina layer 198 is disposed against a bottom surface of the alumina layer 196.

The EM shield 240 is disposed on top surface of the alumina layer 198 and is electrically coupled to electrode 226 which is further electrically coupled to electrical ground. The EM shield 240 is provided to shield the exhaust gas sensor 182 from undesirable electromagnetic noise.

The alumina layer 200 is disposed against a bottom surface of the alumina layer 198. The alumina layer 202 is disposed against a bottom surface of the alumina layer 200. The alumina layer 204 is disposed against a bottom surface of the alumina layer 202. The alumina layer 206 is disposed against a bottom surface of the alumina layer 204. Further, the alumina layer 208 is disposed against a bottom surface of the alumina layer 206.

The heater coil 242 is disposed of a top surface of the alumina layer 208 and is provided to generate heat in response to receiving a voltage from the voltage source 184. In particular, the heater coil 242 is electrically coupled to electrodes 244, 248 which are further electrically coupled to the voltage source 184.

The impedance measurement circuit 186 is provided to measure an impedance of the exhaust gas sensor 182. The impedance measurement circuit 186 has a substantially similar structure as the impedance measurement circuit 16 described above.

The controller 187 is provided to measure a voltage between the NOx sensing electrode 220 and the platinum electrode 224 in order to calculate a NOx concentration. The controller 187 is further provided to measure a voltage between the ammonia sensing electrode 222 and the platinum electrode 224 in order to calculate an ammonia concentration. The controller 187 is further provided to induce the impedance measurement circuit 186 to measure an impedance of the exhaust gas sensor 182 and to calculate a temperature of the exhaust gas sensor 182 based on the impedance. The controller 187 also generates a control voltage to induce the voltage source 184 to output a desired power level, based on the calculated temperature, in order to cause the heater coil 242 to maintain the exhaust gas sensor 182 within a desired temperature range.

Referring to Figure 8, a method for determining concentrations of NOx and ammonia in exhaust gases, and a temperature of the exhaust gas sensor 182 will now be explained.

At step 260, the controller 187 measures a first voltage between the NOx sensing electrode 220 and the platinum electrode 224 that is indicative of a concentration of NOx. The controller 187 calculates a NOx concentration value based on the first voltage.

At step 262, the controller 187 measures a second voltage between the ammonia sensing electrode 222 and the platinum electrode 224 that is indicative of a concentration of ammonia. The controller 187 calculates an ammonia concentration value based on the second voltage.

At step 264, the controller 187, after measuring the first voltage and the second voltage, induces the impedance measurement circuit 186 to measure an impedance between the impedance sensing electrode 232 and the platinum electrode 224 that is indicative of a temperature of the exhaust gas sensor 182.

At step 266, the controller 187 calculates a temperature of the exhaust gas sensor 182 based on the measured impedance between the impedance sensing electrode 232 and the platinum electrode 224.

At step 268, the controller 187 generates a control voltage to induce the voltage source 184 to output a desired power level, based on the calculated temperature, in order to cause the heater coil 242 to maintain the exhaust gas sensor 182 within a desired temperature range. After step 268, the method returns to step 260.

Referring to Figure 9, an exhaust gas monitoring system 280 for determining concentrations of NOx and ammonia, and a temperature of the exhaust gas sensor 282 is illustrated. The exhaust gas monitoring system 280 includes the exhaust gas sensor 282, a voltage source 284, an impedance measurement circuit 286, and a controller 287. The primary difference between the exhaust gas sensor 282 and the exhaust gas sensor 181 is the location of the EM shield.

The exhaust gas sensor 282 is provided to generate a voltage indicative of a detected NOx concentration in exhaust gases communicating with the exhaust gas sensor 282. The exhaust gas sensor 282 is further configured to generate another voltage indicative of a detected ammonia concentration in exhaust gases communicating with the exhaust gas sensor 282. Further, an impedance of a zirconia layer of the exhaust gas sensor 282 is indicative of a temperature of the exhaust gas sensor 282. The exhaust gas sensor 282 includes a zirconia layer 290, alumina layers 292, 294, 296, 298, 300, 302, 304, 306, 308, a NOx sensing electrode 310, an ammonia sensing electrode 312, a platinum pad 313, a platinum reference electrode 314, electrodes 316, 318, 320, 322, an EM shield 330, a heater coil 332, and electrodes 334, 336 and 338.

The NOx sensing electrode 310 extends through the alumina layer 294 and contacts the zirconia layer 290. The NOx sensing electrode 310 is further electrically coupled to the electrode 318 on the alumina layer 292. The ammonia sensing electrode 312 extends through the platinum pad 313 and the alumina layer 294 and contacts the zirconia layer 290. The ammonia sensing electrode 312 is further electrically coupled to the electrode 320 on the alumina layer 292. The reference platinum electrode 314 is disposed on a bottom surface of the zirconia layer 290 and is further electrically coupled to the electrode 316. The electrode 316 is electrically coupled to electrical ground. The platinum electrode 314 communicates with an aperture (not shown) in the zirconia layer 290 that further communicates with exhaust gases. Further, the NOx sensing electrode 310 and the ammonia sensing electrode 312 communicate with the exhaust gases. The combination of the NOx sensing electrode 310, the zirconia layer 290, and the platinum electrode 314 form an electrochemical cell which generates a voltage indicative of a concentration of NOx in exhaust gases communicating with the exhaust gas sensor 282. The combination of the ammonia sensing electrode 312, the zirconia layer 290, and the platinum electrode 314 form an electrochemical cell which generates a voltage indicative of a concentration of ammonia in exhaust gases communicating with the exhaust gas sensor 282. In one exemplary embodiment, the NOx sensing electrode 310 is constructed from TbCrO3 (doped with 5% MgO). Of course in alternative embodiments, other materials known to those skilled in the art could be utilized to construct the NOx sensing electrode 310. In one exemplary embodiment, the ammonia sensing electrode 312 is constructed from BiVO4 (doped with 5% MgO). Of course in alternative embodiments, other materials known to those skilled in the art could be utilized to construct the ammonia sensing electrode 312.

The alumina layer 292 is disposed adjacent a top side of the alumina layer 290. The alumina layer 292 has electrodes 316, 318, 320 disposed thereon. The electrode 316 is further electrically coupled to the impedance measurement circuit 286. The electrodes 318, 320 are electrically coupled to the controller 287.

The zirconia layer 290 is disposed against a bottom surface of the alumina layer 294. In one exemplary embodiment, the zirconia layer 290 is constructed from a combination of zirconia, alumina, and ytteria. Of course, other combinations of zirconia and other materials know to those skilled in the art could be utilized in other alternative embodiments. The platinum temperature sensing electrode 322 is disposed on a top surface of the zirconia layer 290 and is electrically coupled to electrode 336. The EM shield 330 is disposed on a top surface of the alumina layer 296 and is electrically coupled to the platinum electrode 314 which is further electrically coupled to electrical ground.

The alumina layer 296 is disposed against a bottom surface of the zirconia layer 290. The alumina layer 298 is disposed against a bottom surface of the alumina layer 296. The alumina layer 300 is disposed against a bottom surface of the alumina layer 298. The alumina layer 302 is disposed against a bottom surface of the alumina layer 300. The alumina layer 304 is disposed against a bottom surface of the alumina layer 302. The alumina layer 306 is disposed against a bottom surface of the alumina layer 304. Further, the alumina layer 308 is disposed against a bottom surface of the alumina layer 306.

The heater coil 332 is disposed of a top surface of the alumina layer 308 and is provided to generate heat in response to receiving a voltage from the voltage source 284. In particular, the heater coil 332 is electrically coupled to electrodes 334, 338 which are further electrically coupled to the voltage source 284.

The impedance measurement circuit 286 is provided to measure an impedance of the exhaust gas sensor 282. The impedance measurement circuit 286 has a substantially similar structure as the impedance measurement circuit 16 described above.

The controller 287 is provided to measure a voltage between the NOx sensing electrode 310 and the platinum reference electrode 314 in order to calculate a NOx concentration. The controller 287 is further provided to measure a voltage between the ammonia sensing electrode 312 and the platinum electrode 314 in order to calculate an ammonia concentration. The controller 287 is further provided to induce the impedance measurement circuit 286 to measure an impedance of the exhaust gas sensor 282 and to calculate a temperature of the exhaust gas sensor 282 based on the impedance. The controller 287 also generates a control voltage to induce the voltage source 284 to output a desired power level, based on the calculated temperature, in order to cause the heater coil 332 to maintain the exhaust gas sensor 282 within a desired temperature range.

The exhaust gas sensors and methods for measuring concentrations of NOx and ammonia and temperatures of the sensors represent a substantial improvement over other sensors and methods. In particular, the exhaust gas sensors and methods provide a technical effect of only utilizing one zirconia layer therein for generating signals indicative of a concentration of NOx and ammonia, and having an impedance indicative of a temperature of the exhaust gas sensors.

The above-described methods can be embodied in the form of computer-implemented software algorithms and apparatuses for practicing those processes. In an exemplary embodiment, the method is embodied in computer program code executed by one or more elements. The present method may be embodied in the form of computer program code containing instructions stored in tangible media, such as floppy diskettes, CD-ROMs, hard drives, flash memory, or any other computer-readable storage medium, wherein, when the computer program code is loaded into and executed by a computer, the computer becomes an apparatus for practicing the invention.

While the invention has been described with reference to exemplary embodiments, it will be understood by those skilled in the art that various changes may be made and equivalent elements may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiments disclosed for carrying out this invention, but that the invention will include all embodiments falling within the scope of the appended claims. Moreover, the use of the terms first, second, etc. do not denote any order or importance, but rather the terms first, second, etc. are used to distinguish one element from another. Further, the use of the terms a, an, etc. do not denote a limitation of quantity, but rather denote the presence of at least one of the referenced item.

## Claims

1. An exhaust gas sensor (12), comprising:
only one zirconia layer (28) having a first side and a second side opposite the first side;
a NOx sensing electrode (50) coupled to the first side of the zirconia layer (28);
an ammonia sensing electrode (52) coupled to the first side of the zirconia layer (28);
a platinum electrode (54) coupled to the second side of the zirconia layer (28), the platinum electrode (54) being electrically grounded;
the NOx sensing electrode (50), the ammonia sensing electrode (52), and the platinum electrode (54) being configured to communicate with exhaust gases;
wherein a first voltage between the NOx sensing electrode (50) and the platinum electrode (54) is indicative of a concentration of NOx, and a second voltage between the ammonia sensing electrode (52) and the platinum electrode (54) is indicative of a concentration of ammonia; and
wherein an impedance between the NOx sensing electrode (50) and the platinum electrode (54) is indicative of a temperature of the exhaust gas sensor (12).

2. The exhaust gas sensor (12) of claim 1, wherein an impedance between the ammonia sensing electrode (52) and the platinum electrode (54) is also indicative of the temperature of the exhaust gas sensor (12).

3. A method for measuring concentrations of both NOx and ammonia and a temperature utilizing an exhaust gas sensor (12), the exhaust gas sensor (12) having only one zirconia layer (28) having a first side and a second side opposite the first side, a NOx sensing electrode (50) coupled to the first side of the zirconia layer (28), an ammonia sensing electrode (52) coupled to the first side of the zirconia layer (28), and a platinum electrode (54) coupled to the second side of the zirconia layer (28), the platinum electrode (54) being electrically grounded, the NOx sensing electrode (50), the ammonia sensing electrode (52), and the platinum electrode (54) being configured to communicate with exhaust gases, the method comprising:
measuring a first voltage between the NOx sensing electrode (50) and the platinum electrode (54) that is indicative of the concentration of NOx;
measuring a second voltage between the ammonia sensing electrode (52) and the platinum electrode (54) that is indicative of the concentration of ammonia; and
after measuring the first voltage and the second voltage measuring an impedance between the NOx sensing electrode (50) and the platinum electrode (54) that is indicative of the temperature of the exhaust gas sensor (12).

4. A method for measuring concentrations of both NOx and ammonia and a temperature utilizing an exhaust gas sensor (12), the exhaust gas sensor (12) having only one zirconia layer (28) having a first side and a second side opposite the first side, a NOx sensing electrode (50) coupled to the first side of the zirconia layer (28), an ammonia sensing electrode (52) coupled to the first side of the zirconia layer (28), and a platinum electrode (54) coupled to the second side of the zirconia layer (28), the platinum electrode (54) being electrically grounded, the NOx sensing electrode (50), the ammonia sensing electrode (52), and the platinum electrode (54) being configured to communicate with exhaust gases, the method comprising:
measuring a first voltage between the NOx sensing electrode (50) and the platinum electrode (54) that is indicative of the concentration of NOx;
measuring a second voltage between the ammonia sensing electrode (52) and the platinum electrode (54) that is indicative of the concentration of ammonia; and
after measuring the first voltage and the second voltage, measuring an impedance between the ammonia sensing electrode (52) and the platinum electrode (54) that is indicative of the temperature of the exhaust gas sensor.

5. An exhaust gas sensor (182), comprising:
only one zirconia layer (190) having a first side and a second side opposite the first side;
a NOx sensing electrode (220) coupled to the first side of the zirconia layer (190);
an ammonia sensing electrode (222) coupled to the first side of the zirconia layer (190);
a platinum electrode (224) coupled to the second side of the zirconia layer (190), the platinum electrode (224) being electrically grounded;
the NOx sensing electrode (220), the ammonia sensing electrode (222), and the platinum electrode (224) being configured to communicate with exhaust gases;
an impedance sensing electrode coupled to the first side of the zirconia layer (190); and
wherein a first voltage between the NOx sensing electrode (220) and the platinum electrode (224) is indicative of a concentration of NOx, and a second voltage between the ammonia sensing electrode (222) and the platinum electrode (224) is indicative of a concentration of ammonia, and an impedance between the impedance sensing electrode (232) and the platinum electrode (224) is indicative of a temperature of the exhaust gas sensor (182).

6. The exhaust gas sensor (182) of claim 5, further comprising an electromagnetic shield (240) coupled to the second side of the zirconia layer (190), the electromagnetic shield (240) being further electrically coupled to the platinum electrode (224).

7. A method for measuring concentrations of both NOx and ammonia and a temperature utilizing an exhaust gas sensor (182), the exhaust gas sensor (182) having only one zirconia layer (190) having a first side and a second side opposite the first side, a NOx sensing electrode (220) coupled to the first side of the zirconia layer (190), an ammonia sensing electrode (222) coupled to the first side of the zirconia layer (190), a platinum electrode (224) coupled to the second side of the zirconia layer (190), the platinum electrode (224) being electrically grounded, and an impedance sensing electrode coupled to the first side of the zirconia layer (190), the NOx sensing electrode (220), the ammonia sensing electrode (222), and the platinum electrode (224) being configured to communicate with exhaust gases, the method comprising:
measuring a first voltage between the NOx sensing electrode (220) and the platinum electrode (224) that is indicative of the concentration of NOx;
measuring a second voltage between the ammonia sensing electrode (222) and the platinum electrode (224) that is indicative of the concentration of ammonia; and
after measuring the first voltage and the second voltage, measuring an impedance between the impedance sensing electrode (232) and the platinum electrode (224) that is indicative of the temperature of the exhaust gas sensor (182).
